# EUROPEAN PATENT APPLICATION

(11) **EP 4 115 939 A1**
(43) Date of publication of application: **11.01.2023**
(21) Application number: 21382598.7
(22) Date of filing: 05.07.2021
(51) Int. Cl.: A61N 1/04, A61N 1/36, A63F 13/285, G06F 3/01

(54) **MULTI-PAD ELECTRODE AND SYSTEM FOR PROVIDING ELECTROTACTILE STIMULATION**

(71) Applicant: Fundación Tecnalia Research & Innovation, 20009 Donostia - San Sebastián (ES)
(72) Inventor: Keller, Thierry, 20009 San Sebastián - Donostia (GUIPÚZCOA) (ES); Imatz Ojanguren, Eukene, 20009 San Sebastián - Donostia (GUIPÚZCOA) (ES); Isakovic, Milica, 20009 San Sebastián - Donostia (GUIPÚZCOA) (ES); Hernandez Jimenez, Erik, 20009 San Sebastián - Donostia (GUIPÚZCOA) (ES); Strbac, Matija, 20009 San Sebastián - Donostia (GUIPÚZCOA) (ES); Kostic, Milos, 20009 San Sebastián - Donostia (GUIPÚZCOA) (ES)
(74) Representative: Balder IP Law, S.L.

(57) **Abstract**

A multi-pad electrode (10, 20) for providing electrotactile stimulation to a hand of user, comprising: a plurality of activation pads (11) configured to be individually electrically activated, wherein in use of the multi-pad electrode (10, 20), the plurality of activation electrode pads (11) is in contact with at least one of the palm, fingers, thumb and dorsum of the user's hand; a return pad (15) whose size is at least four times larger than the size of each activation pad (11),), wherein in use of the multi-pad electrode (10, 20), the return pad (15) is in contact with one of the palm, fingers, thumb and dorsum of the user's hand. A system (100) for providing electrotactile stimulation to a hand of a user, comprising: a multi-pad electrode (10, 20); and a device (60) for eliciting tactile sensations in the hand of the user, the device (60) comprising: a pulse generator (62) for generating a plurality of electrical pulses for eliciting tactile sensations; and a switching unit (67) for spatio-temporal routing a generated electrical stimulation pulse to a corresponding activation pad (11) of the multi-pad electrode (10, 20).

## Description

### TECHNICAL FIELD

The present invention relates to the field of wearable electrodes for providing physical feedback to the user wearing the electrode. In particular, it relates to wearable electrodes for providing physical feedback to a user in the field of Xtended reality, such as virtual, augmented and multimodal mixed reality, in which tactile electrical stimulation can provide electrotactile sensations for immersive interaction with visualized objects in virtual contact with the human skin.

### STATE OF THE ART

Xtended reality (XR) is a simulated experience applicable to different fields, such as entertainment (e.g. games including video games), education (e.g. medical or military training) and business (e.g. virtual meetings). A user can get into a virtual world by interacting with a VR system. This interaction usually implies transmitting information from the VR system to the user and vice versa. For example, the VR system replies to actions of the user. Currently, there are different VR systems that provide interactivity and certain realistic physical feedback in a virtual environment. This may be achieved, for example, using VR headsets having a head-mounted display or specially designed rooms with multiple large screens, to generate realistic images, sounds and other sensations that simulate a user's physical presence in a virtual environment. In order for a user to enjoy a realistic immersion effect, other types of sensory inputs different from images and audio may be required, such as sensory and force feedback provided through haptic technology.

For example, US10948991 B2 discloses a VR system that provides haptic feedback to users. The haptic feedback is in the form of an electrical stimulus applied by a set of electrodes disposed on a user's limb, to the muscles of the user. The electrodes are preferably provided in pairs to avoid cross-talk, in such a way that a stimulation signal is applied to one of the electrodes of each pair, with the other electrode of the pair acting as ground.

Transmission of realistic tactile sensation to a user has therefore been of high interest in many application fields, such as entertainment, teleoperation, training and education, and assistive technologies for many decades. However, there are many challenges to achieving this aim. The biggest challenge is the inability to produce the stimuli with sufficiently high resolution in the highly sensitive areas, like fingertips or tongue. Tactile displays provide feedback to the user by stimulating the skin mechanically (e.g., vibration motors) or electrically. They are commonly used as feedback interfaces in virtual reality applications (B. Serrano, R. Baños and C. Botella, "Virtual reality and stimulation of touch and smell for inducing relaxation: A randomized controlled trial," Computers in Human Behavior, vol. 55, pp. 1-8, 2016), teleoperation (D. A. Kontarinis and R. D. Howe, "Tactile display of vibratory information in teleoperation and virtual environments.," Presence: Teleoperators & Virtual Environments, vol. 4, no. 4, pp. 387-402, 1995), mobile environment communication (E. Hoggan, S. A. Brewster and J. Johnston, "Investigating the effectiveness of tactile feedback for mobile touchscreens.," in Proceedings of the SIGCHI conference on Human factors in computing systems, 2008) as well as in prosthetics, to provide sensory information from the missing limb (artificial touch) (S. Dosen, M. Markovic, M. Strbac, M. Belić, V. Kojic, G. Bijelic, T. Keller and D. Farina, "Multichannel electrotactile feedback with spatial and mixed coding for closed-loop control of grasping force in hand prostheses," IEEE Transactions on Neural Systems and Rehabilitation Engineering, vol. 25, no. 3, pp. 183- 195, 2017).

Vibrotactile stimulation is much more popular in contemporary consumer devices, as vibration motors are easy to apply, and there are many applications in which sending only the most basic information (e.g., notifications) is sufficient (L. A. Jones and N. B. Sarter, "Tactile displays: Guidance for their design and application," Human factors, vol. 50, no. 1, pp. 90-111, 2008). However, vibration motors are not suitable for conveying complex information (e.g., pressure distribution over the palm of the hand), due to their size, sound and energy consumption. In addition, the stimulation parameters are coupled through resonance behaviour (mass spring system) (M. Azadi and L. A. Jones, "Vibrotactile actuators: Effect of load and body site on performance," in 2014 IEEE Haptics Symposium (HAPTICS), 2014).

With advancement of printed electronics, electrotactile systems have received more attention in the research systems. Successful use of high-density matrix electrodes for eliciting high-fidelity sensations has been reported by several groups (V. Yem, R. Okazaki and H. Kajimoto, "FinGAR: combination of electrical and mechanical stimulation for high-fidelity tactile presentation," in ACM SIGGRAPH 2016 Emerging Technologies, 2016), providing significant amount of evidence that this approach has the potential to unlock the possibilities of tactile communication. In electrotactile systems, low-intensity electrical current is delivered to the skin to activate cutaneous nerve fibres and elicit tactile sensations. Tactile sensations can be a) soft touch sense, which enables one to detect fine detail regarding the location, size, shape, and texture of an object; b) light pressure, e.g. from an object placed against the skin; c) strong pressure, e.g. from an impact force; d) vibrations; e) heat, cold; and f) pain; occurring through activation of the following skin receptors: 1) Meissner's Corpuscle - These sensory receptors are located nearest to the skin's surface. When these corpuscles are deformed by pressure, they send neural signals to the brain. These receptors perceive fine touch, pressure, and vibrations; 2) Merkel's Disk - These receptors are nerve endings located at the bottom of the epidermis and in hair follicles. They send signals to the brain that decode the features of touched objects such as their texture and temperature. They are extremely sensitive to pain; 3) Ruffini Ending - These receptors perceive any kind of stretching of the skin. They also detect temperature and pressure; 4) Pacinian Corpuscle - Detects deep pressure sensations. These receptors play a large role in proprioception; 5) Krause End Bulb - These are sensitive to the cold. In fact, they only activate when touching something with a temperature below 20 degrees; and 6) Free Never Endings - They are non-specialized nerve cell endings. They respond to all stimuli - pressure, temperature, pain, texture, etc.

The advantages of electrotactile interfaces are simple structure, low-power consumption and low cost, since there are no moving mechanical elements. Furthermore, unlike force or vibration, the sensation generated is not masked by movements, making them suitable for Xtended reality applications. They can integrate a large number of tactile electrodes and allow independent modulation of stimulation parameters. Besides, the electrodes can be customized in shape and size.

However, the state of the art is limited to scenarios where basic sensations like vibration or pressure are used in a monotonal manner to present gross tactile information, like object roughness or hardness. In addition, the stimulation is typically localized to a small area, such as, a fingertip, which limits the type of mechanical interaction that can be transmitted through the interface.

US10437334B2 discloses a method and wearable apparatus for interactive physiological and technological synchronization of a user with a virtual environment. The wearable apparatus comprises electrodes for supplying electrical feedback pulses to nerve endings. In order to obtain a realistic sensing system, electrodes are supplied with pulses of various amplitude, frequency and stress using cascaded distribution across a set of electrode contact points. The wearable apparatus is for example a glove-manipulator that allows for fitting the electrodes tight against a body.

However, electrode positioning and maintaining temporal stability of delivered sensations are unsolved challenges. Consequently, conventional electrode interfaces have not managed to provide the user with such realistic simulated experience as expected. Such limitations have prevented further research and development of dependent applications, such as use of tactile feedback in XR to deliver natural interfaces and improve sensibility, or applications in psychological treatments.

Therefore, there is a need to develop new multi-pad electrode interfaces which overcome the drawbacks of conventional ones.

### DESCRIPTION OF THE INVENTION

The present invention overcomes the drawbacks of conventional multi-pad electrodes for electrotractile stimulation and systems comprising these electrodes, by providing a multi-pad electrode having a distribution of pads which minimizes the tactile sensation in areas of the user's body different from the area on which the pad receiving the electrical pulse, is disposed.

The proposed multi-pad electrode may be integrated in a wearable garment, such as a glove, in such a way that the user wearing it can get electrotactile sensations in different parts of his/her hand, such as fingers, thumb, palm or dorsum, because electrode pads comprised in the multi-pad electrode are in contact with different points in the user's hand, thus being able to provide electrotactile stimulation. Electrotactile stimulation is done through electrical pulses generated by a device for providing electrotactile stimulation, which can be attached to the glove, integrated therein, or connected thereto. This device may receive remote instructions, for example from a XR controller. At the device, electrical pulses for eliciting tactile sensations are generated, and the electrical pulses are routed to activation pads of the multi-pad electrode. Controlled spatial and temporal current flow between each activation pad and a return pad is allowed. The multi-pad electrode and system are specially designed for providing electrotactile stimulation to healthy users, meaning for example users not having a nervous system disorder.

The electrical pulses generated for electrotactile stimulation can be current controlled electrical pulses or voltage controlled electrical pulses. Both voltage and current controlled pulses can establish a closed current circuit requiring at least one activation electrode pad and one return electrode pad. In practice, a plurality of activation electrodes (also referred to as pads) are used in order to activate tactile sensations in different areas of a user's hand. In embodiments of the invention, a single return electrode pad can be used.

In a first aspect of the invention, a multi-pad electrode for providing electrotactile stimulation to a hand of user is provided. The multi-pad electrode comprises: a plurality of activation pads configured to be individually electrically activated, wherein in use of the multi-pad electrode, the plurality of activation electrode pads is in contact with at least one of the palm, fingers, thumb and dorsum of the user's hand; and a return pad whose size is at least four times larger than the size of each activation pad, wherein in use of the multi-pad electrode, the return pad is in contact with one of the palm, fingers, thumb and dorsum of the user's hand.

The proposed configuration enables activation precisely under the activation pad skin receptors (skin receptors or nerves under a selected activation pad), but does not activate skin receptors or nerves under the return electrode pad.

In embodiments of the invention, the return pad is located in a position on the multi-pad electrode such that, in use of the multi-pad electrode, the return pad is at a central area of the palm of the user's hand.

In embodiments of the invention, the multi-pad electrode further comprises a plurality of secondary return pads, the secondary return pads are connected to the return pad through a conductive path.

In embodiments of the invention, the multi-pad electrode is implemented on a flexible substrate.

In embodiments of the invention, the multi-pad electrode is implemented on a stretchable substrate.

In embodiments of the invention, the size of the return pad is at least 6 times larger than the size of each activation pad, or at least 8 times larger, 10 times larger, or 14 times larger, or 18 times larger.

In a second aspect of the invention, a glove made of a textile material is provided, the glove having integrated therein the multi-pad electrode of the first aspect of the invention.

In a third aspect of the invention, a system for providing electrotactile stimulation to a hand of a user, is provided. The system comprises a multi-pad electrode according to the first aspect of the invention; and a device for eliciting tactile sensations in the hand of the user, the device comprising: a pulse generator for generating a plurality of electrical pulses for eliciting tactile sensations; and a switching unit for spatio-temporal routing a generated electrical stimulation pulse to a corresponding activation pad of the multi-pad electrode.

In embodiments of the invention, the pulse generator is current-controlled.

In embodiments of the invention, wherein when a generated electrical stimulation pulse is routed to a corresponding activation pad of the multi-pad electrode, a close current path is established between the activation pad and the return pad.

In a fourth aspect of the invention, an Xtended Reality system is provided, comprising: the system for providing electrotactile stimulation to a hand of a user, of the third aspect of the invention; a sensing system for capturing the position and orientation of system for providing electrotactile stimulation; and a controller for, from the position and orientation of the system of the third aspect of the invention captured by the sensing system, providing instructions to the system of the third aspect of the invention for providing electrotactile stimulation.

The invention provides an electrotactile electrode which is of simple structure, low-power consumption and low cost, since it does not require moving mechanical elements. Furthermore, the sensation generated is not masked by movements, making it suitable for Xtended reality applications. It can integrate a large number of tactile electrode pads and allows independent modulation of stimulation parameters (e.g., location, intensity and frequency). Therefore, it provides tactile feedback with high resolution and/or communicate multiple variables simultaneously. The electrode pads can be customized in shape and size. The design can be adapted to a specific application and body location.

In addition, unlike prior art devices, where basic sensations like vibration or pressure are used in a monotonal manner to present gross tactile information, like object roughness or hardness, in the proposed system a large collection of sensations is achieved. In addition, unlike conventional devices, in which the stimulation is typically localized to a small area, such as, a fingertip, which limits the type of mechanical interaction that can be transmitted through the interface, the proposed system provides stimulation to different hand areas, such as fingers, thumb, palm and dorsum of the hand. Furthermore, electrode size, shape and arrangement are optimized with respect to perceived sensations, to the physical characteristics of the skin surface and to the complexity of the touch information that are aimed to deliver.

The electrode of the invention overcomes the relatively poor accuracy of the simulated sensation achieved by conventional devices (caused, for example, by influence of an electrical pulse intended for a specific electrode contact, on others electrode contacts (for which that specific electrical pulse is not intended)).

Additional advantages and features of the invention will become apparent from the detailed description that follows and will be particularly pointed out in the appended claims.

### BRIEF DESCRIPTION OF THE DRAWINGS

To complete the description and in order to provide for a better understanding of the invention, a set of drawings is provided. Said drawings form an integral part of the description and illustrate an embodiment of the invention, which should not be interpreted as restricting the scope of the invention, but just as an example of how the invention can be carried out. The drawings comprise the following figures:
Figures 1A-1B show different views of a system in the form of a glove for providing electrotactile stimulation according to embodiments of the invention.
Figure 2 shows exemplary charge balance pulses that may be supplied to a multi-pad electrode according to embodiments of the invention.
Figure 3A shows a detailed scheme of a multi-pad electrode according to embodiments of the invention, in which the electrode pads and corresponding conductive paths are depicted.
Figure 3B shows an enlarged view of the part of the multi-pad electrode designed to be placed on the palm of a user's hand.
Figure 4 shows an alternative multi-pad electrode according to embodiments of the invention.
Figure 5 shows a device for generating electrical pulses for exciting selected electrode pads of a multi-pad electrode according to embodiments of the invention.
Figure 6 shows an exemplary hardware element implementing a pulse generator according to embodiments of the invention.
Figure 7 shows a block diagram of an Xtended reality system for providing feedback to a user wearing a system having a multi-pad electrode integrated therein.

### DESCRIPTION OF A WAY OF CARRYING OUT THE INVENTION

The following description is not to be taken in a limiting sense but is given solely for the purpose of describing the broad principles of the invention. Next embodiments of the invention will be described by way of example, with reference to the above-mentioned drawings, showing apparatuses and results according to the invention.

Next, different embodiments of multi-pad electrodes and systems for providing electrotactile feedback, according to the invention, are disclosed.

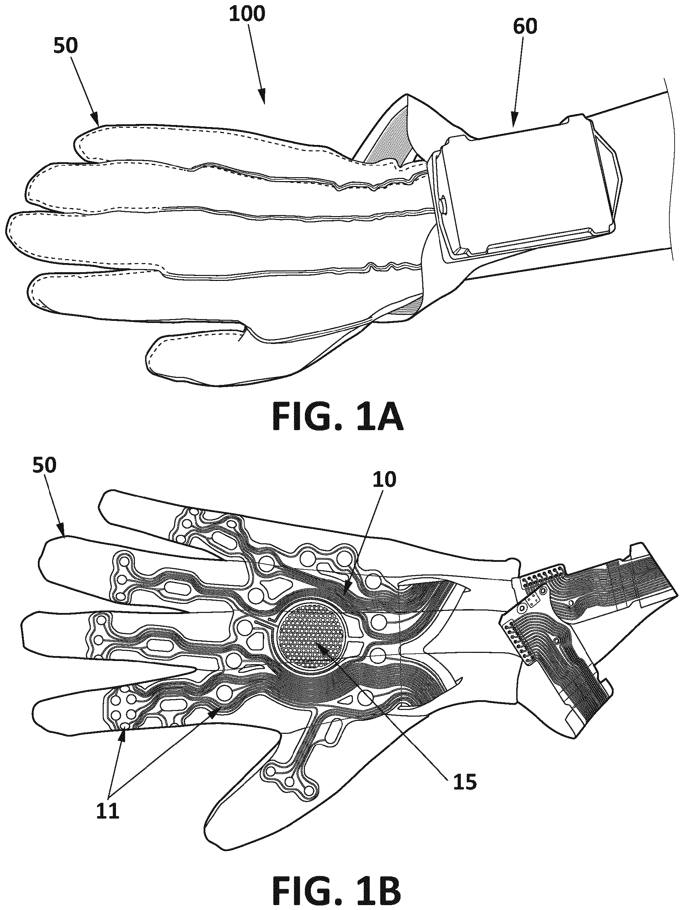

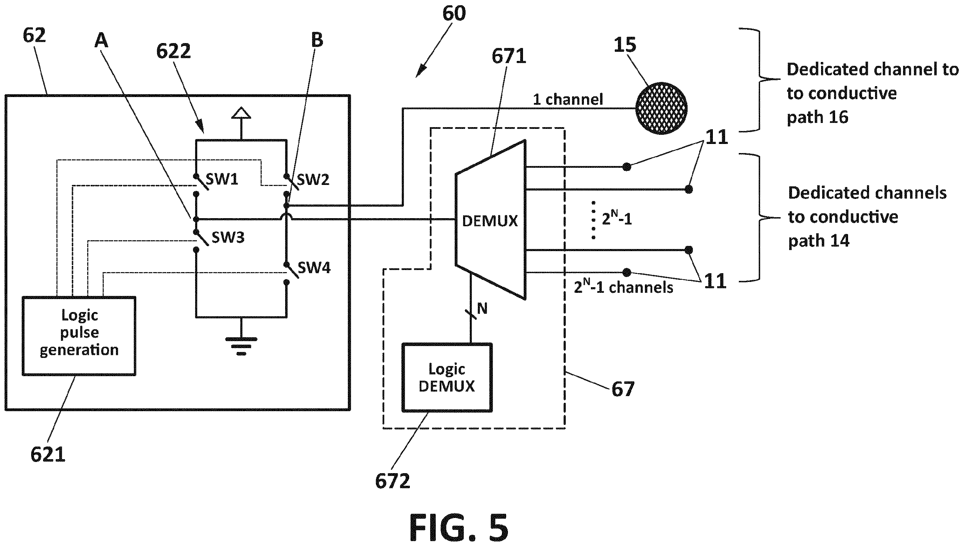

Figures 1A-1B show two views of a system 100 for providing electrotactile stimulation. System 100 is embodied as a glove 50 to be worn by a user, in such a way that the user can get electrotactile sensations in different parts of his/her hand, such as fingers, palm or dorsum. Figure 1A shows the dorsum side of the glove 50, that is to say, the part of the glove 50 to be disposed on the user's dorsum. In turn, Figure 1B shows the palm side of the glove 50, that is to say, the part of the glove 50 to be disposed on the user's palm. When electrotactile stimulation is provided in response to the system 100 being close to or in contact with an object, for example in a XR application, the electrotactile stimulation is referred to as electrotactile feedback. Electrotactile stimulation aims at activating skin receptors. Electrotactile stimulation is done through electrical pulses generated by a device 60 for providing electrotactile stimulation, which can be attached to the glove 50, integrated therein, or connected thereto. As shown in Figure 1B, the part of the glove 50 disposed on the user's palm incorporates a multi-pad electrode 10 according to an embodiment of the invention. The multi-pad electrode 10 is integrated into the textile glove 50, in particular into the palm side of the glove 50. This way, when a person wears the glove 50, the electrode pads comprised in the multi-pad electrode 10 are in contact with the palm of the user's hand, thus being able to provide electrotactile stimulation. At device 60, electrical pulses for eliciting tactile sensations are generated, and the electrical pulses are routed to the pads of the multi-pad electrode 10. The device 60 is described later with reference to Figure 5. Device 60 allows controlled -spatial and temporal- current flow between activation pads and return pad(s).

The electrical pulses generated for electrotactile stimulation are preferably current-controlled electrical pulses. For example, electrical pulse trains of around 0.2-10mA for a duration of 60-1000µs per pulse at a repletion frequency of 2-500Hz, may be used. These are reasonable ranges for electrotactile stimulation for healthy users (meaning for example users not having a nervous system disorder). It is remarked that, in general, amplitudes lower than 0.2mA produce no sensation, while amplitudes above 10mA produce additional effects like muscle contraction in addition to electrotactile effects. Frequencies lower than 2Hz are felt as separated tactile cues (single pin pricks not as vibration), while frequencies higher than 500Hz do not generate additional nerve action potiential in the same nerve fiber as the refractory period is about 2ms. Pulse durations lower than 60 µs are not perceived unless the amplitude is in the range of 10^{th} of mA. Durations longer than 1000ms impact the generation of high repetition frequencies. Current-controlled electrical pulses are also referred to as current regulated pulses. Alternatively, voltage-controlled electrical pulses (also referred to as voltage regulated pulses) may be used, but they are less preferred because they are less precise due to the fact that the skin impedance is highly variable and highly dependent on the actual current. Both voltage and current pulses establish a closed current circuit requiring at least one activation electrode and one return electrode. In practice, a plurality of activation electrodes (also referred to as pads) are used in order to activate tactile sensations in different areas of a user's hand. In embodiments of the invention, a single return electrode pad can be used.

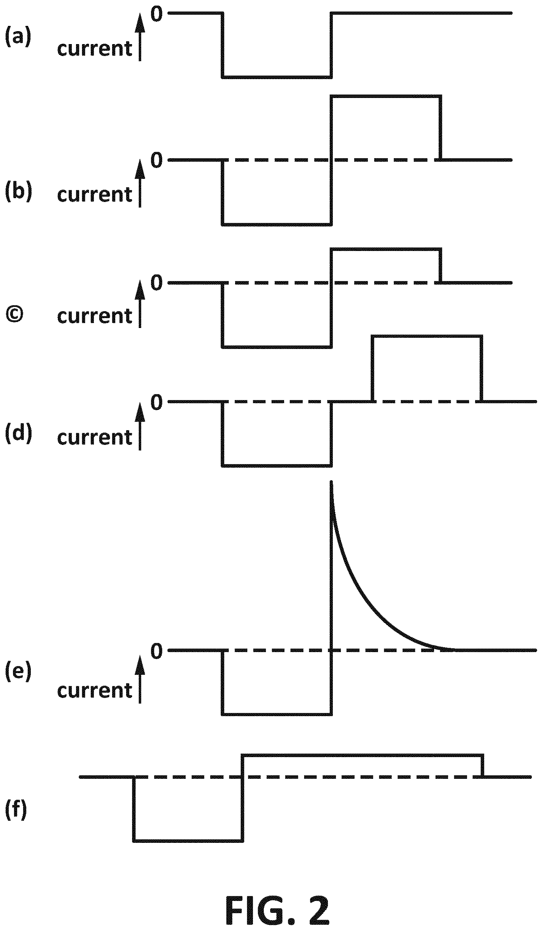

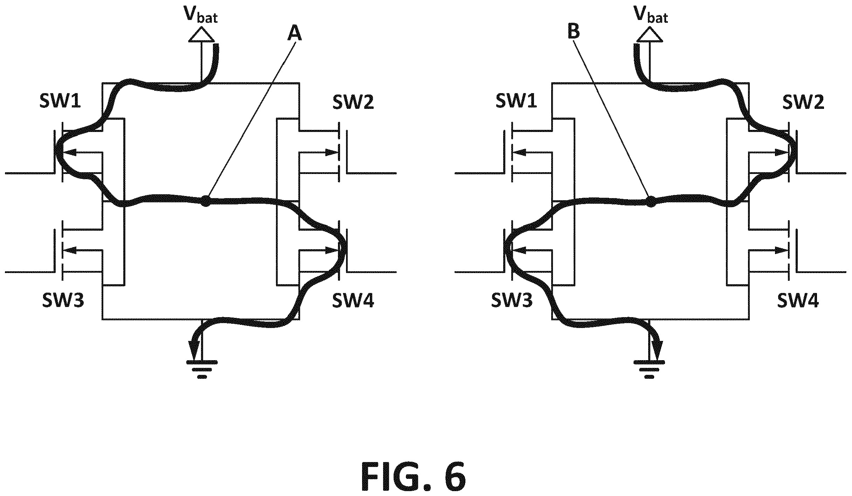

For reasons of minimizing adverse effects on skin and underlying tissue layers it is recommended to use charge balanced currents when using pulse trains. Charge balanced pulses usually comprise an activation pulse and a charge compensation pulse. In other words, charge balanced pulses are the concatenation of an activation pulse and a charge compensation pulse. The activation pulse is usually the negative pulse (or more precisely, the negative part of the pulse), while the charge compensation pulse is usually the positive pulse (or more precisely, the positive part of the pulse). Figure 2 shows exemplary charge balance pulses that may be used to provide electrotactile stimulation through a multi-pad electrode 10 according to the invention: monophasic pulse (a), charge balanced biphasic pulse (b), charge imbalanced biphasic pulse (c), charge balanced biphasic with delay pulse (d), charge balanced biphasic with fast reversal pulse (e), and charge balanced biphasic with slow reversal pulse (f). Although the monophasic pulse (a) only has activation pulse, but no charge compensation pulse, it is technically possible to generate both pulses, for example with a H-bridge (Figure 6).

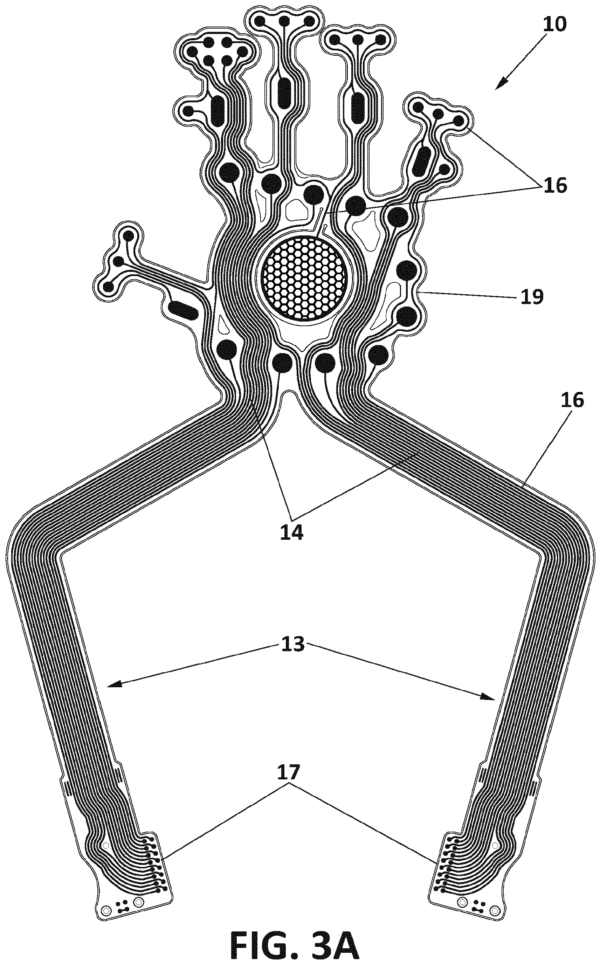

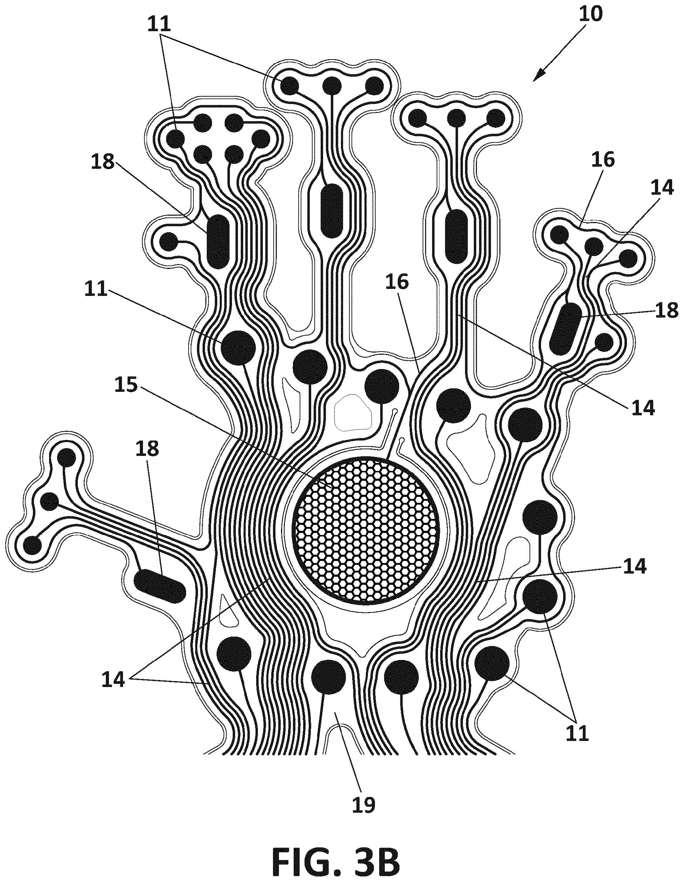

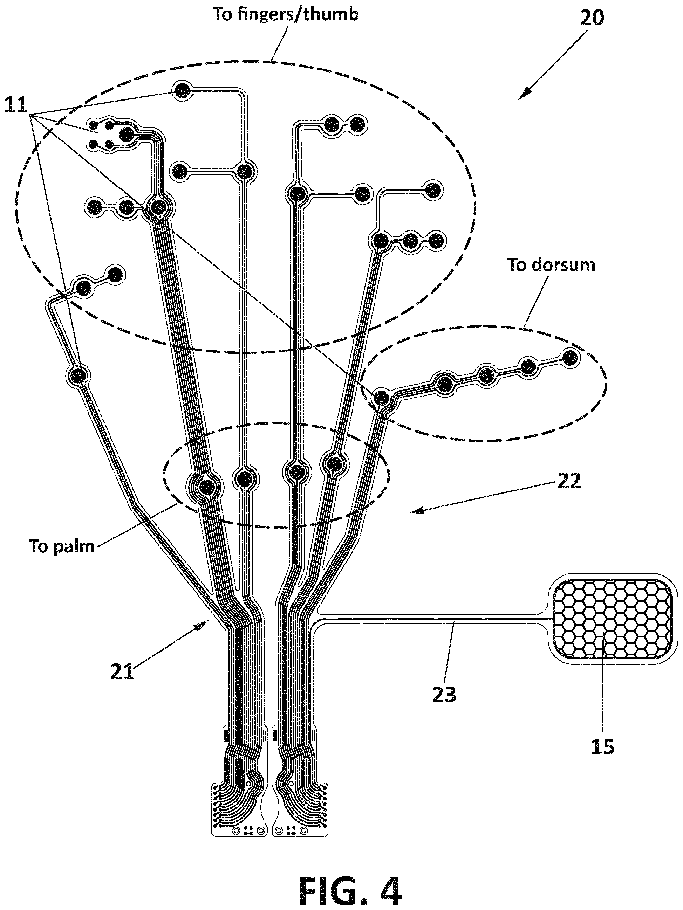

Figure 3A shows a detailed scheme of the multi-pad electrode 10 of Figure 1B, intended for use on a hand of a user, in particular on the palm of a hand. An enlarged view of the portion of the electrode 10 to be located on the user's palm is shown in Figure 3B. Figure 4 shows a detailed scheme of an alternative multi-pad electrode 20 to be used on a hand of a user. Multi-pad electrode 10, 20 is usually made of a flexible substrate 19. The flexible substrate 19 can be made of a textile material, a nano-cellulose-based material or a polymeric material, for example. The material is preferably stretchable, for example a stretchable polymeric material, such as Thermoplastic Polyurethane (TPU). Multi-pad electrodes made with these materials, in particular textile and stretchable polymeric ones, have a great advantage over conventional electrodes made of non-stretchable materials due to higher usability, comfort and adherence to the human body, which are of great importance in wearable devices applications. Improvements in production and design of electrical stimulation systems have reached a stage where systems with a large number of individual electrode actuators can be embedded in a sleeve, vest or a glove. The multi-pad electrode 10, 20 can be integrated into a textile glove 50, for example by heat transfer of the conductive (and optionally dielectric) inks, which are preferably stretchable inks. The glove textile is preferably stretchable, so that it tightens the electrode 10, 20 and therefore electrode pads 11, 15, 18 to the user's skin.

Electrode 10, 20 comprises a plurality of electrode pads that may be distributed in an arbitrary configuration. Activation pads may be disposed on specific areas of the substrate so that different areas of the fingers, thumb, palm and/or dorsum of the hand are excited. Electrode pads and corresponding conductive paths are made of a conductive material, preferably a biocompatible material at least for parts of the pads in contact with the user. For example, electrode pads may be made of stainless steel, gold, titanium, carbon, silver or combinations thereof. Preferably, during the manufacturing process of the multi-pad electrode, on top of the electrode pads a coating layer is placed, such as a conductive coating, as for example graphite or polymeric coating, such as poly(3,4-ethylenedioxythiophene) polystyrene sulfonate (PEDOT:PSS). On top of the conductive paths (wires) there is usually a non-conductive coating for preventing electrical contact with the user's skin. The activation pads and return pad(s) and associated wiring can be attached to or integrated on the flexible substrate using different techniques, such as screen printed, plated, stitched, embroidered, knitted or use any textile or additive printing technology. Conductive printing technologies on flexible polymer films or onto textile (or hybrid) substrates or nano-cellulose-based substrates, which foresee to maintain the stretchability of the wearable system (glove) despite the high number and density of electrodes and interconnections, are preferred. As a matter of example, electrode pads may be printed by means of stretchable ink, such as biocompatible inks. In the manufacturing process, conductive inks are used. Optionally dielectric inks may also be used. Examples of inks are silver ink, graphite ink or a combination of inks of different conductive materials. The process of manufacturing the multi-pad electrode and the integration of the electrode pads in the substrate are out of the scope of the present invention.

Electrode 10 has electrode pads 11, 15, 18 of which a plurality of them are activation pads 11 and one or more of them are return pads 15, 18. The electrode 10 allows controlled -spatial and temporal- current flow between activation pads 11 and return pad(s) 15, 18. A single return pad 15 is enough. However, in embodiments of the invention, as shown in Figures 3A-3B, there may be additional return pads 18. In charge balanced pulsed currents, an activation pulse (that is to say, the activation component of the charged balanced pulse, which is usually the negative component of the pulse) has the effect of activating excitable tissues under the activation electrode pad 11 (usually working as cathode) to which the pulse is addressed, during the period the current depolarizes the nerve resting potential. Conversely, the charge compensation pulse (that is to say, the charge compensation component of the charged balanced pulse, which is usually the positive component of the pulse) hyperpolarizes the excitable tissue. Therefore, the charge compensation pulse needs to be administered with a well-designed electrode system when a precise localisation and type of sensation is aimed. However, on the return electrode 15, 18 the same happens in reverse: the charge compensation pulse has the effect of potentially activating excitable tissues under the return electrode pad 15 (usually working as anode) during the period the current depolarizes the nerve resting potential, and the activation pulse hyperpolarizes the excitable tissue. Therefore, in order to allow precise localisation of electrotactile stimulation, there should be an activation of the skin receptors under the selected activation electrode pad 11, but no activation of skin receptors under the return electrode 15, 18. This is achieved in the multi-pad electrode of the present invention. There may be activation pads 11 at positions in the multi-pad electrode 10 corresponding to different points on the fingers, thumb, palm and dorsum of the user's hand. In the shown electrode 10, there are activation pads 11 on the palm, fingers (both at the palm and the dorsum side) and thumb (both at the palm and the dorsum side). It is remarked that the multi-pad electrode 10 is made of a flexible substrate 19 that adapts to the hand's shape, so that most of its surface matches the surface of the user's palm, while certain portions can be bended, wrapping for example the user's fingers and thumb, so that outer finger/thumb electrode pads are disposed on the user's fingers/thumb, at the dorsum side.

Inventors have surprisingly noticed that, in order to design a multi-channel electrotactile system that is capable of activating precisely under the activation pad skin receptors (skin receptors or nerves under a selected activation pad), but not activating skin receptors or nerves under the return electrode pad, the multi-pad electrode must have a substantially larger return electrode pad 15 than each of the activation electrode pads 11. The size (area) of the return pad 15 must be at least four (4) times larger than the size (area) of each activation pad 11. For example, the area of the return pad 15 can be six (6) times larger than the area of the activation pad 11, or eight (8) times larger, or ten (10) times larger, or fourteen (14) times larger, or eighteen (18) times larger, or twenty (20) times larger. In the embodiment shown in Figure 3B, the area of the return pad 15 is approx. 10 times larger than the area of the activation pads 11. Inventors have observed that, when the size of the return pad 15 is four times larger than the size of each activation pad 11, it imitates quite well the fact that a hyperpolarizing pulse generates an action potential under a pad when the stimulation intensity is chosen 4 times higher than the excitation threshold of a depolarizing pulse at the same location.

The location in the multi-pad electrode 10, 20 of the return electrode pad 15 must also be properly selected. For example, the return pad 15 should not be placed close to (in the vicinity of) large nerve fibers close to the surface of the hand, in order to prevent the activation of nerves or receptors by the charge compensation pulse of same shape and intensity as the activation pulse. Inventors have surprisingly found out that the best location of the return pad 15 is on the palm of the hand (that is to say, on the area of substrate 19, 29 designed to be in contact with the user's palm). More particularly, the best location of the return pad 15 has been determined to be the central area of the palm of the hand. As shown in Figure 3B, there is a plurality of activation pads 11 disposed also on the palm, but in the peripheral area thereof, that is to say, for example surrounding the return pad 15. Thus, a plurality of activation pads 11 selectively activates in such a configuration the skin receptors under the active activation pads 11 without spreading the current to other locations (causing non-desired sensation). In particular, it has been observed that the placement of the return pad 15 on other places different from the palm, such as certain areas of dorsum or wrist, may lead to feel sensations in non-desired areas of the hand, such as in the fingers, namely when the return pad 15 is placed on or close to the wrist, on the dorsal part of the hand, or at locations on the forearm and shoulder regions. Alternatively, the return pad 15 may be disposed on the fingers, thumb or dorsum of the hand. In Figures 3A-3B, activation pads 11 have been designed to be placed on the user's palm, thumb and different points of fingers, such as fingertips and/or different phalanges.

In preferred embodiments, the combination of size ratio between return pad 15 and activation pad 11 (a ratio of at least 4:1) and the proper selection of location of the return pad 15 on the central area of the user's palm has shown optimal results.

In embodiments of the invention, in addition to the main return pad 15, other secondary return pads 18 are used. This is shown for example in the embodiment of Figures 3A-3B. These secondary return electrode pads 18 are connected to the main one 15 through conductive path 16. It has been observed that secondary return pads 18 contribute to have the required return current path in situations in which the main return pad 15 does not make good contact with the user's skin. For example, when due to the movement of the user's hand, the contact between the main return pad 15 and the user's skin is poor. The secondary return pads 18 thus contribute to increase the sensation on the desired area of the skin/nerves and to suitably close the current path. For example, there may be one additional return pad 18 per finger (and thumb), as shown in Figures 3A-3B. In embodiments of the invention, the size of the secondary return pads 18 is not so large as the size of the main one 15. In embodiments of the invention, the size of the secondary return pads 18 is around four times the size of the activation pads 11.

Activation pads 11 and return pads 15, 18 may have any shape. They may for example be round, oval or with polygonal shape having substantially rounded corners. In Figures 3A-3B the activation pads 11 are circular (they have the shape of a circle), but they could have other shapes. In Figures 3A-3B the secondary return pads 18 are oval, but they could have other shapes. For example, all the pads 11, 18 could have the same shape, for example circular or oval. Sharp edges are less recommended because they could lead to locally higher current densities due to edge effects.

On the substrate 19 there are conductive paths 14, 24 for connecting each activation pad 11 with corresponding outputs of the demultiplexing stage of device 60. Stimulation electronics based on demultiplexing circuits allow spatio-temporal distribution of electrical pulses over a plurality of activation pads 11 through the conductive paths 14, 24. In the present case, there are as many independently controlled activation channels as the total number of activation pads 11. As a matter of example, in the multi-pad electrode 10 of Figure 3A-3B there are 31 activation pads 11, and therefore 31 independently controlled activation channels. However, there is one single conductive path 16 connecting the return pad 15 with the corresponding output of the device 60. This conductive path 16 carries the charge compensation pulse. This has been proven important to support high-fidelity electrotactile stimulation. The activation electrode pads 11 can be individually selected (by means of a demultiplexing stage of device 60) and receive individual stimulation parameters (for example amplitude, pulse width and frequency). Demultiplexing can also be set such that a plurality of activation pads 11 receives the same activation pulse, which current then is divided among the activation pads 11 according to their overall impedance between the activation pads and the return pads.

Figure 3A shows two branches 13 made of flexible substrate. These two branches 13 are made of the same portion of substrate 19 of which the multi-pad electrode 10 is made. However, these two branches 13 do not have electrode pads 11, 15, 18 integrated therein, but only carry the wires 14 (electrical paths or conductive paths) from each activation pad 11, plus the single wire 16 (conductive path) from the return pad or pads 15, 18, to device 60. Conductive paths 14, 16 connect a respective pad (activation pad 11 or return pad 15) with a respective termination point 17 disposed at the distal end of the branches 13 (the distal end being the end opposite to the end closer to the electrode pads). For example, conductive paths 14, 16 may be made of stainless steel, gold, titanium, carbon, silver or combinations thereof, for example made with conductive ink. At the shown distal end, output pins of the device 60 (at termination point 17) are electrically connected to the conductive paths 14, 16 of the electrode 10, 20 and therefore electrically connected to respective electrode pads 11, 15, 18.

Figure 4 shows an alternative embodiment of a multi-pad electrode 20. In this case the electrode 20 is made up of two independent portions 21, 22 of flexible substrate 19. The two independent portions 21, 22 provide freedom to adapt the electrode pads 11, 15 to a best position on the user's hand. In this embodiment, certain activation pads 11 are designed to be in contact with the user's palm, some others with the user's dorsum and some others with the user's fingers and thumb, including fingertips and/or different phalanges. Thus, electrotactile stimulation is directed to palm, dorsum and fingers/thumb of the user. In this embodiment, there is a single return electrode 15, built in one of the two portions 21, 22, to which it is connected through a thin portion 23 of substrate. An optimal position of the return electrode 15 on the central area of the user's palm can thus be achieved.

Next, a device 60 as illustrated in Figures 1A-1B, for generating electrical pulses for eliciting tactile sensations and for providing them to a multi-pad electrode 10, 20, is described with reference to Figure 5. Device 60 is also referred to as stimulator. In Figure 5, 2^{N}-1 channels connect the stimulator 60 with the 2^{N}-1 conductive paths 14, 16 of the multi-pad electrode 10, 20. In other words, each activation pad 11 of electrode 10, 20 is connected to the stimulator 60 by means of a dedicated channel 14. An additional dedicated channel connects the stimulator 60 with the one or more return electrodes 15, 18. In other words, irrespective of the number of return pads (one single main return pad 15, or a main return pad 15 plus secondary ones 18), there is one single wired path 16 (a single channel). In Figure 5 the number of channels for the activation pads is a power of 2 minus 1 (2^{N}-1), but in fact the number of activation pads is not necessarily constraint to a power of 2. These channels (2^{N}-1 channels in Figure 5) are the interface between the stimulator 60 and the multi-pad electrode 10, 20.

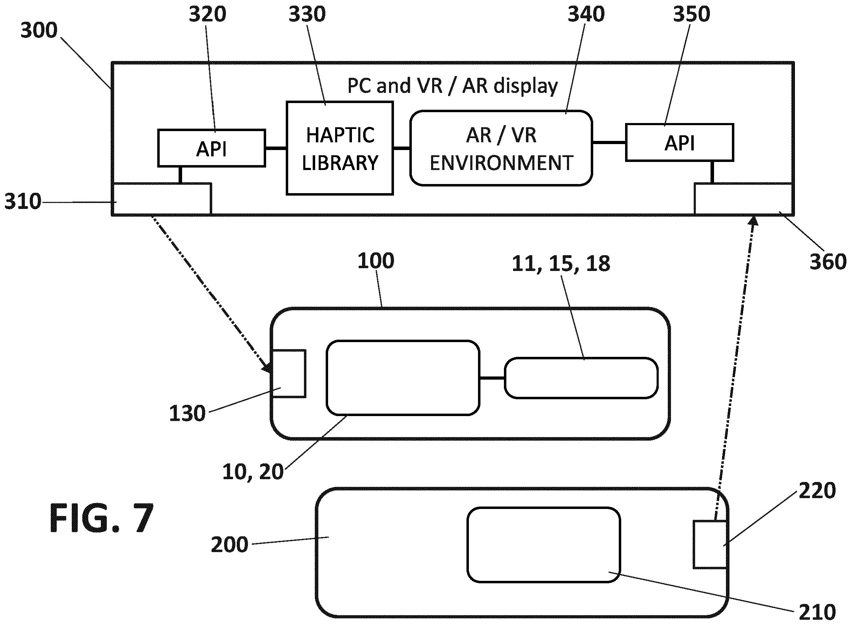

Device 60 has a pulse generator 62 for generating a stimulation signal (an electrical pulse) and a switching unit 67 for routing the generated electrical pulse to corresponding activation pads 11. Pulse generator 62 is comprised of a hardware element 622 and a logic element 621. The logic element 621 is implemented as computer instructions embedded in processing means of device 62, such as a microprocessor. The logic element 621 (logic pulse generation) generates pulses according to instructions, for example received from an external computer 300 as explained with reference to Figure 7. The electrical pulses generated by the logic element 621 are preferably electrical current pulses. The generated electrical pulses carry individual stimulation parameters, such as amplitude, pulse width and frequency. For example, electrical current pulse trains of around 0.2-10.0mA for a duration of 60-1000µs per pulse at a repletion frequency of 5-500Hz, may be generated. The intensity of trains may be modulated to imitate the intensity of touch, impact force, pressure, heat or pain, among others. The different stimulation parameters enable the activation of a broad range of tactile sensations, such as, but not limiting: soft touch sense, which enables to detect fine details regarding location, size, shape and/or texture of an object; ranges of light pressure, e.g. from an object placed against the skin to strong pressure, e.g. from an impact force; vibrations; sensation of heat; and pain. As a matter of example, a stimulation frequency of around 3 Hz provides the feeling of vibration, while a stimulation frequency of around 100 Hz provides the feeling of touch/pressure.

In a particular embodiment, the hardware element 622 is implemented as an H-bridge comprising four switching elements SW1-SW4, for example implemented with transistors, such as FET transistors. Logic element 621 has four outputs, each of which is connected to a corresponding switching element SW1-SW4, so that the open/close state of each switching element SW1-SW4is controlled. In Figure 5, switches SW2 and SW3 are closed (and SW1 and SW4 are open) to generate the activation pulse, and switches SW1 and SW4 are closed (and SW2 and SW3 are open) to generate the compensation pulse. All switches SW1-SW4 are open when no current flows between the pads. The point A between switches SW1 and SW3 is connected to the activation pads switching unit 67. The point B between switches SW2 and SW4 is directly connected to the dedicated channel in turn connected to conductive path 16 in the multi-pad electrode 10, 20, which is the conductive path connected to the return pad 15 (or return pads 15, 18).

Figure 6 shows an example of H-bridge 622, in which the four switching elements SW1-SW4 are implemented with corresponding transistors. In Figure 6, the current path is emphasized: When switches SW1 and SW4 are closed and SW2 and SW3 are open, the current flows as depicted in Figure 6 (left). Point A as shown in Figure 5 is also shown in Figure 6 (left). When switches SW1 and SW4 are open and SW2 and SW3 are closed, the current flows as depicted in Figure 6 (right). Point B as shown in Figure 5 is also shown in Figure 6 (right). This enables to change polarity of the generated pulses. The channel connected to the return pad 15 (or return pads 15, 18) is necessary to close the circulation of current within the multi-pad electrode 10, 20, required when charge balanced pulses are used, which is the case here. The return pad 15 takes some current, but does not excite the tissue under it. Instead, the current flows back to the H-bridge 622 from the return pad(s) through the single channel (path 16).

Regarding the switching unit 67, it has a hardware element (switching circuit) 671 and a logic one 672. Switching circuit 671, for example implemented as a demultiplexer, selects, among all the activation pads 11, the activation pad to which the generated pulse must be routed at each specific time instant. Switching circuit 671 is controlled by control logic 672, which controls the switching circuit 671 through N inputs. The logic element 672 is implemented as computer instructions embedded in processing means of device 67, such as a microprocessor. N inputs are capable of controlling up to 2^{N}-1 outputs of switching circuit 671. Each pad 11 of the multi-pad electrode 10, 20 is connected to the stimulator 60 through a direct lead or channel 14. Therefore, the number of activation pads 11 of the multi-pad electrode 10, 20 is equal to the number of physical leads (channels) 14. In alternative embodiments of the switching unit 67, the switching circuit 671 can be implemented with more than one demultiplexer, for example with two demultiplexers, each of them dealing with half of the dedicated channels connected to the activation pads. In alternative embodiments, one demultiplexor connects point B to all pads and another demultiplexor connects point A to all pads. This way, every pad can act as activation pad or as return pad.

The system 100 for providing electrotactile stimulation of the present invention can be used in Xtended Reality (XR) applications. In this case, the system 100 is combined with a Virtual Reality (VR), Augmented Reality (AR) or mixed reality system that includes a sensing system 200 for capturing the position and orientation of system 100 and a controller 300 comprising a processing unit. Controller 300 can be for example embodied as a personal computer or computer system. This is described with reference to Figure 7. An example of a sensing system 200 for capturing the position and orientation of system 100 is a glove system that can be worn over the glove 50 implementing system 100. System 200 (outer glove) may comprise different sensor units 210 which produce data representative of an orientation and position (and optionally velocity and/or acceleration of the hand wearing systems 100, 200. Non-limiting examples of these sensor units 210 are kinematic sensors, IMUs, compasses, accelerometers, inertial measurement units, strain gauges and video cameras. System 200 provides the output data to the controller 300 through a wireless interface 220 comprising transmitting/receiving means. Alternatively, system 200 may be implemented in a different form from a glove to be worn over glove 50, provided that system 200 enables to assess the position and orientation of glove 50 (system 100). For example, it may be implemented as a garment, different from a glove, worn by the user or attached thereto. Sensing system 200 is out of the scope of the present invention.

The controller 300 receives, by means of a corresponding wireless interface 360, the output data from system 200. The controller 300 uses this data to estimate the position and orientation of the hand of the user wearing the system 200 (and therefore, the position and orientation of system 100). This is done at module 350, which may be implemented as an API. Once the position and orientation of system 200 is known, and therefore is known the position and orientation of system 100, the controller 300 sends instructions to system 100. With these instructions, the device 60 (stimulator) generates electrical pulses for eliciting tactile sensations to be delivered to selected activation pads 11 of the multi-pad electrode 10, 20, which are synchronized with the current position and orientation of the glove 50 (system 100) and therefore system 200. In other words, instructions refer to electrotactile feedback to be delivered to the user's hand. The controller 300 provides these instructions to system 100 (to device 60) through a wireless interface 310 comprising transmitting/receiving means. Instructions are in turn received at system 100 at a corresponding wireless interface 130. Non-limiting examples or wireless communication technologies that may be used are Bluetooth or WiFi, among others. Therefore, system 100 comprises a wireless interface 130 comprising transmitting/receiving means, not shown in Figure 1A-1B, for communication with controller 300. Therefore, controller 300 commands the current intensity to be generated by the pulse generator 62 and the parameters (such as amplitude, time duration and stimulation frequency) of the electrical pulses to be produced by the pulse generator 62. This is done by means of instructions sent from the controller 300 to the logic pulse generation 621. It also commands (sends instructions to) the switching circuitry 67 (in particular, to the logic demux 672) for selecting the specific discrete activation pads 11 to which the generated pulse(s) must be distributed.

The controller 300 builds instructions to be delivered to system 100 from data received from system 200, using modules 330, 330. Module 340 represents an AR/VR environment, which is a description of the virtual world the user is watching. For example, module 340 may use the software called Unity, which is a XR software development environment for generating in real-time synchronized virtual objects. Module 330 is a haptic library which translates interactions between an object and the real-time localized hand of the user, into tactile sensations to be applied at precise locations with system 100. In other words, modules 340 and 330 synchronize the real environment with the virtual one. In the context of the present invention, real-time refers to the generation of tactile sensations at the right position(s) within a maximum amount of delay of 100ms, such as within 80ms, or 60ms, or 30ms or 10ms.

A prototype including a multi-pad electrode 100 (Figure 3A-3B) integrated in a glove 50 (Figure 1A-1B) has been manufactured and tested. In the prototype, the size of the return pad 15 is 10 times larger than the size of each activation pad 11. The main return pad 15 has been located at the central area of the palm of the hand, as shown in Figure 1B. Activation pads 11 and secondary return pads 18 were located as shown in Figure 1B. Successfully experienced sensations were light touch, rain drops, pressures, impact collisions of the hand with objects, penetrations into liquids, vibration of parts or the hand, e.g. holding a drilling machine, stings and touch of hot objects. Such accurate sensations are obtained as a consequence of selecting a return electrode 15 having a total surface of at least four times the surface of each activation pad 11 and locating the return electrode pad 15 at the central area of the palm of the user's hand. This is not achieved in conventional electrotactile systems, wherein return current paths are not specifically considered.

A possible application of the multi-pad electrode and system of the invention can be in the treatment of certain disorders, such as phobia. Let's imagine a person having a disruptive fear of spiders. The phobia can significantly lower the quality of his/her life, with physical sensations such as sweating, nervousness, anxiety, dizziness and nausea. The treatment can be based on a VR graded exposure therapy, involving the patient exposing herself, with the help of the VR and system of the invention, to spiders in a series of experiences of graduated difficulty. The patient may start with looking at spider images with varying number and sizes of insects in the VR environment, and using the system of the invention (for example a glove implementing the multi-pad electrode) to touch other elements in the environment in order to get a deeper sense of presence. From session to session, the VR spiders will be progressively closer to the patient. Eventually, he/she will be able to touch and hold a spider in the VR environment and feel it with the inventive system. By the end of the treatment, the patient will be able to face real spiders without experiencing anxiety and get out outside without concerns.

Another application of the multi-pad electrode and system of the invention can be in field of gaming. Let's imagine a software developer and a passionate gamer, user of modern consoles including motion tracking, haptic controllers and AR/VR headsets. His/her new gadget may be the glove electrode system of the present invention, which will enable him/her to have a much more natural interaction with virtual environment than with any conventional hand-held controller. Its physic-based interaction algorithms perfectly mimic the user's real hand movements captured through kinematic sensors, allowing the user intuitive multi-DOF control of his/her avatar and give him/her natural-like tactile feedback. Importantly, this feedback is not a simple buzzing users used to get from conventional gaming controllers with an integrated vibrator. Instead, the glove elicits spatially distributed feeling of touch, simulating natural tactile interaction with virtual objects, and thereby, creating the feeling of immersiveness, thus really feeling the virtual world.

Another application of the multi-pad electrode and system of the invention can be in field of VR training. Let's think of a student in BTS (advanced vocational training certificate), involved in practical session, consisting of checking the seal on the drain plug of a plane (A380) and replace if necessary. As the campus has no real unit from this series, this session is realized in VR environment. The delicate handling is carried out "blind" because the plug is placed behind the distribution circuit. Thanks to the glove electrode system of the invention, the student can place his/her arm behind a virtual distribution circuit and feel the mechanical constraint on his/her hand.

In this text, the term "comprises" and its derivations (such as "comprising", etc.) should not be understood in an excluding sense, that is, these terms should not be interpreted as excluding the possibility that what is described and defined may include further elements, steps, etc.

In the context of the present disclosure, the term "approximately" and terms of its family (such as "approximate", etc.) should be understood as indicating values very near to those which accompany the aforementioned term. That is to say, a deviation within reasonable limits from an exact value should be accepted, because a skilled person in the art will understand that such a deviation from the values indicated is inevitable due to measurement inaccuracies, etc. The same applies to the terms "about", "around" and "substantially".

The invention is obviously not limited to the specific embodiment(s) described herein, but also encompasses any variations that may be considered by any person skilled in the art (for example, as regards the choice of materials, dimensions, components, configuration, etc.), within the general scope of the invention as defined in the claims.

## Claims

1. A multi-pad electrode (10, 20) for providing electrotactile stimulation to a hand of user, comprising:
a plurality of activation pads (11) configured to be individually electrically activated, wherein in use of the multi-pad electrode (10, 20), the plurality of activation electrode pads (11) is in contact with at least one of the palm, fingers, thumb and dorsum of the user's hand;
a return pad (15) whose size is at least four times larger than the size of each activation pad (11), wherein in use of the multi-pad electrode (10, 20), the return pad (15) is in contact with one of the palm, fingers, thumb and dorsum of the user's hand.

2. The multi-pad electrode (10, 20) of claim 1, wherein the return pad (15) is located in a position on the multi-pad electrode (10, 20) such that, in use of the multi-pad electrode (10, 20), the return pad (15) is at a central area of the palm of the user's hand.

3. The multi-pad electrode (10, 20) of any one of claims 1-2, further comprising a plurality of secondary return pads (18), the secondary return pads (18) being connected to the return pad (15) through a conductive path (16).

4. The multi-pad electrode (10, 20) of any one of claims 1-3, which is implemented on a flexible substrate (19).

5. The multi-pad electrode (10, 20) of any one of claims 1-4, which is implemented on a stretchable substrate (19).

6. The multi-pad electrode (10, 20) of any one of claims 4-5, which is produced by screenprinting of one or more inks on the substrate (19).

7. The multi-pad electrode (10, 20) of any one of claims 1-6, wherein the size of the return pad (15) is at least 6 times larger than the size of each activation pad (11), or at least 8 times larger, 10 times larger, or 14 times larger, or 18 times larger.

8. A glove (50) made of a textile material, the glove (50) having integrated therein the multi-pad electrode (10, 20) of any one of claims 1-7.

9. A system (100) for providing electrotactile stimulation to a hand of a user, comprising:
a multi-pad electrode (10, 20) according to any one of claims 1-7; and
a device (60) for eliciting tactile sensations in the hand of the user, the device (60) comprising: a pulse generator (62) for generating a plurality of electrical pulses for eliciting tactile sensations; and a switching unit (67) for spatio-temporal routing a generated electrical stimulation pulse to a corresponding activation pad (11) of the multi-pad electrode (10, 20).

10. The system (100) of claim 9, wherein the pulse generator (62) is current-controlled.

11. The system (100) of either claim 9 or 10, wherein when a generated electrical stimulation pulse is routed to a corresponding activation pad (11) of the multi-pad electrode (10, 20), a close current path is established between the activation pad (11) and the return pad (15).

12. An Xtended Reality system, comprising:
the system (100) for providing electrotactile stimulation to a hand of a user, of any one of claims 9-11;
a sensing system (200) for capturing the position and orientation of system (100) for providing electrotactile stimulation; and
a controller (300) for, from the position and orientation of system (100) captured by the sensing system (200), providing instructions to the system (100) for providing electrotactile stimulation.
